# EUROPEAN PATENT APPLICATION

(11) **EP 3 677 257 A1**
(43) Date of publication of application: **08.07.2020**
(21) Application number: 20150059.2
(22) Date of filing: 02.01.2020
(51) Int. Cl.: A61K 31/015, A61K 31/12, A61K 31/165, A61K 31/426, A61K 31/5375, A61P 17/00, A61P 17/06, A61P 21/00, A61P 29/00

(54) **METHOD FOR TREATING AN INFLAMMATORY DISORDER WITH A SUBSTITUTED PHENYL-PROPENAL MOIETY**

(30) Priority: 04.01.2019 US 201962788573 P
(71) Applicant: Allianz Pharmascience Ltd, 10682 Taipei (TW)
(72) Inventor: CHAN, HARDY W., REDWOOD CITY, CA California 94065 (US)
(74) Representative: Fuchs Patentanwälte Partnerschaft mbB

(57) **Abstract**

The invention provides a method for treating an inflammatory disorder relating an inflammatory cytokine overexpression in a subject in need of such treatment, comprising administrating to said subject a compound with (substituted phenyl)-propenal moiety.

## Description

### FIELD OF THE INVENTION

This invention relates to a treating method, more specifically, the present invention provides a method for treating an inflammatory disorder with compounds with at least one (substituted phenyl)-propenal moiety. This invention further relates to a pharmaceutical composition comprising the compounds for use in a method for treating an inflammatory disorder relating to an inflammatory cytokine overexpression in a subject in need of such treatment.

### BACKGROUND OF THE INVENTION

It is well known that certain natural products may possess therapeutic effects, which has lead to their use in the treatment and prevention of human diseases across many cultures (e.g., Chinese herbal medicines and many other folk medicines). The effectiveness of such treatments has lead the pharmaceutical industry to seek and isolate active compounds from these natural products and develop the active ingredients as therapeutic or prophylactic drugs for the treatment and prevention of a variety of diseases or medical conditions. Thus many commonly used pharmaceuticals have been developed or have arisen from natural products. However, compounds isolated from natural products are known to play certain physiological function(s) in its native host; whereas their therapeutic effects against human diseases are not readily apparent. Historically, such therapeutic treatments were derived merely by accumulated experiences or "trial and error" in humans. Moreover, because such compounds were not initially created for use in humans, the compounds in their native form are frequently not in the most optimal form, both in structure as well as efficacy, to treat human diseases. However, today's modern chemistry technology, including analytical and synthetic chemistries, together with the advances in medicinal biology have made it possible for one to dissect a chemical structure and localize a "pharmacophore" (a core structure that is essential for the therapeutic activity) within a compound such as one isolated from a natural product; furthermore, these new techniques allow one to synthesize new compounds, based on the structure of a pharmacophore, that possess optimal or even better therapeutic efficacy.

Compound curcumin (existing as a major pigment in a turmeric plant) and many of its analogs have been reported to possess numerous biological activities *in vitro,* such as, anti-oxidant, anti-inflammatory, anti-tumor, and antiangiogenesis activities; but neither curcumin nor its analogues have been developed into a therapeutic drug to treat human diseases. This indicates curcumin in its native form is probably not an optimal molecule for development into a therapeutic drug.

Psoriasis is an immune related chronic inflammatory skin disease that has impact on the life quality of 2%-3% of the global population. Psoriasis is typically associated with red, scaly, raised plaques, and a marked thickening of the epidermis induced by inflammation with enhanced keratinocyte proliferation, leukocyte infiltrates in the epidermis and dermis. This disease can be caused by various external and intrinsic factors including microbial infections, skin injure, environment, weather, stress, immune disorders, and genetics. Leukocyte infiltrates in psoriatic lesions mainly contain dendritic cells, macrophages, neutrophils, and T cells. Dendritic cells generate multiple proinflammatory cytokines, including TNF-α, IL-1β, IL-6, and IL-23, that promote the development of psoriasis. TNF-α is a potent proinflammatory stimulus that activates IL-23 production in DCs. IL-1β can promote IL-17 secretion from Th17 cells. IL-6 protects cutaneous T cells from Treg suppression and promotes Th17 participation in inflammation. Together, these immune cells and cytokines promote the inflammatory responses underlying the development of psoriatic lesions. The major mechanism of psoriasis is primarily mediated by the aberrant induction of T cells and dendritic cells (DCs) with Th1/Th17 immune response. Dendritic cells play a role in sensing self-derived nucleic acids that lead to pathogenic inflammatory responses in autoimmune disorders. Therefore, DCs are considered a target in the treatment of many autoimmune disorders, including psoriasis. An effective treatment is needed.

### SUMMARY OF THE INVENTION

The invention is to provide a method and/or a pharmaceutical composition for treating an inflammatory disorder relating an inflammatory cytokine overexpression in a subject in need of such treatment, comprising administrating to said subject a pharmaceutical composition comprising an effective amount of a compound according to formula V or VIII and optionally a pharmaceutically acceptable carrier or excipient; 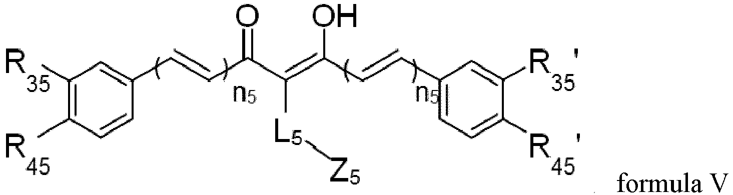 wherein in formula V:
each "ns" is independently 1, 2, or 3;
R₃₅, R₄₅, R₃₅, and R₄₅ are independently selected from the group consisting of -H, - OH, and -OCH₃;
L₅-Z₅ is absent; or
L₅-Z₅ exists, and L₅ is selected from the group consisting of a C0-C8 alkylene, an unsaturated alkenylene, and alkynl when Z₅ is absent;
Z₅ is selected from the group consisting of -H, -OH, an aromatic ring, a cycloalkyl, -COR₁₅, -CO₂R₁₅, -CONR₁₅R₂₅, -NR₁₅R₂₅, -C(X₅)_{3;}
R₁₅ and R₂₅ are independently selected from the group consisting of -H, -CH₃, and -C₂H₅; and
X₅ is a halogen atom selected from the group consisting of -F, -Cl, and -Br; 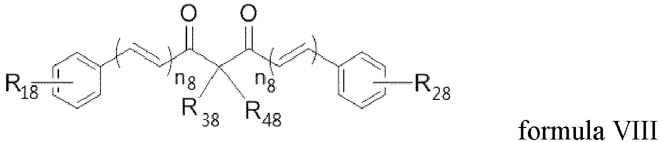
R₁₈ and R₂₈ are mono- or di-substituted groups and independently selected from the group consisting of a methoxy group, a hydroxyl group, and an alkyl sulfonyl group;
R₃₈ is selected from the group consisting of 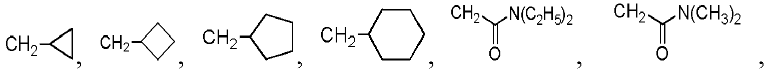 (CH₂)₃CH₃, 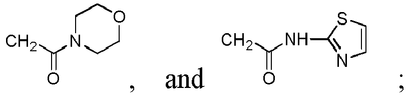 and
R₄₈ is selected from the group consisting of CH₃, H, F and Cl; and
n₈ is 1 or 2;
wherein the inflammatory cytokine is selected from the group consisting of INF-α, IL-1β, IL-17A, IL-22 and IL-31.

The present invention is described in detail in the following sections. Other characteristics, purposes and advantages of the present invention can be found in the detailed description and claims.

### BRIEF DESCRIPTION OF THE DRAWINGS

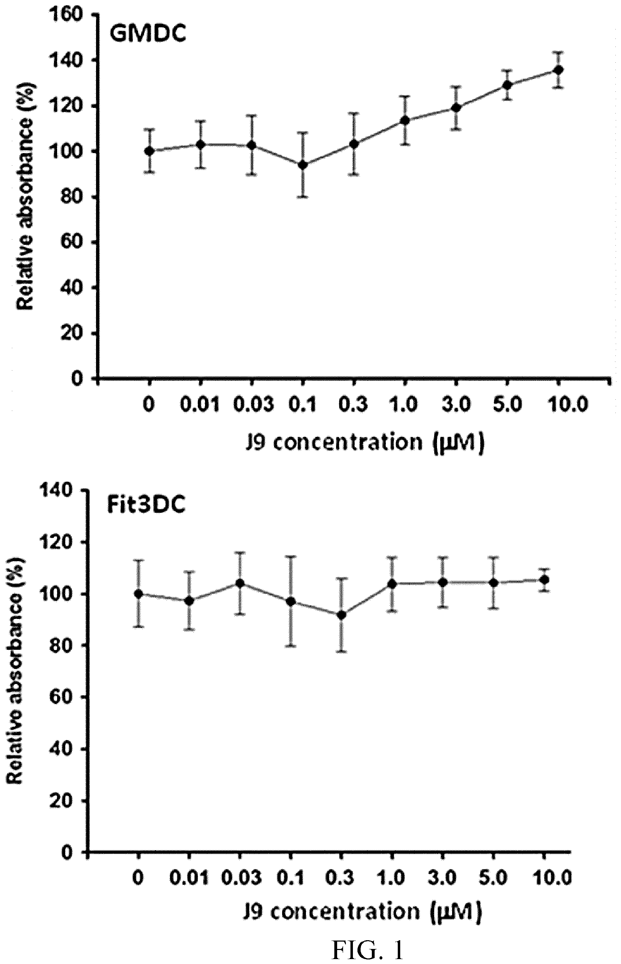

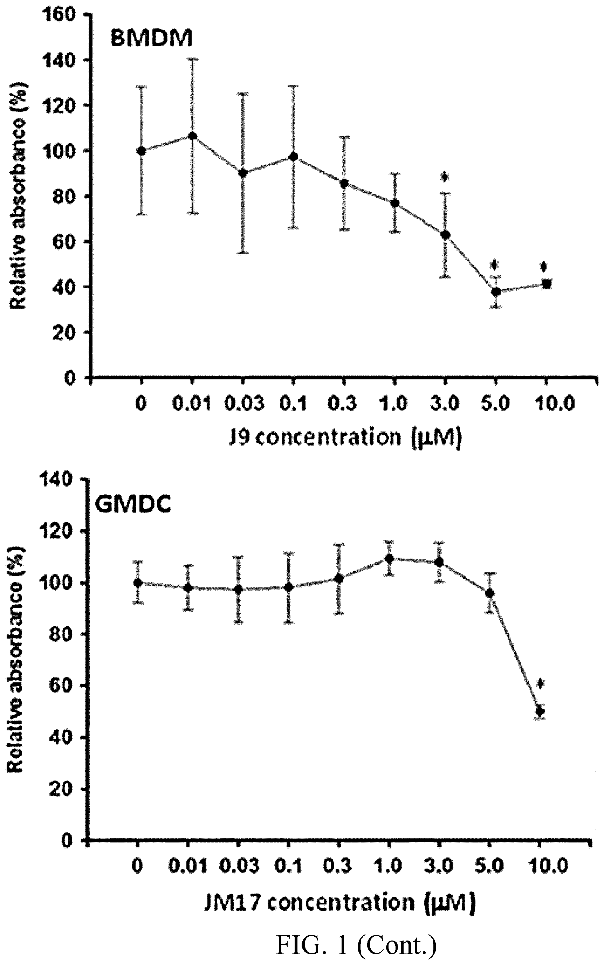

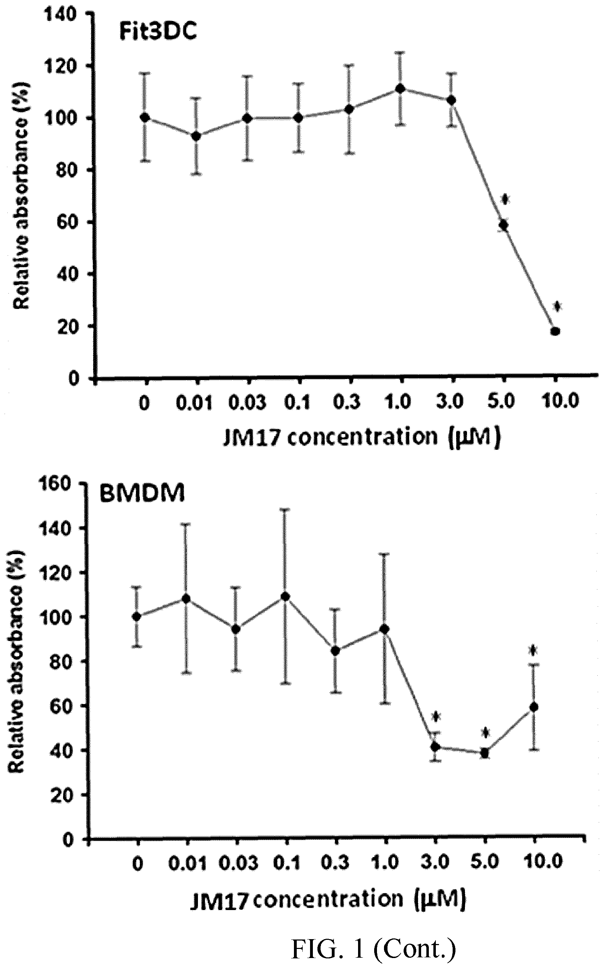

FIG. 1 shows the results of cytotoxicity of ASC-J9 (J9) and ASC-JM17 (JM17) on mouse dendritic cells and macrophages. Cells were treated with different concentration of ASC-J9 and ASC-JM17 for 24 hours. Viability of cells were measured by MTS Assays. *: p < 0.05.

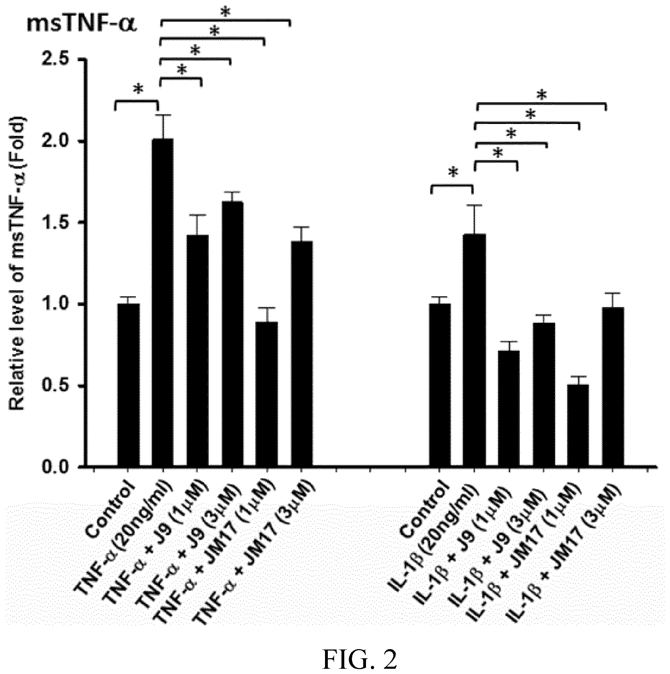

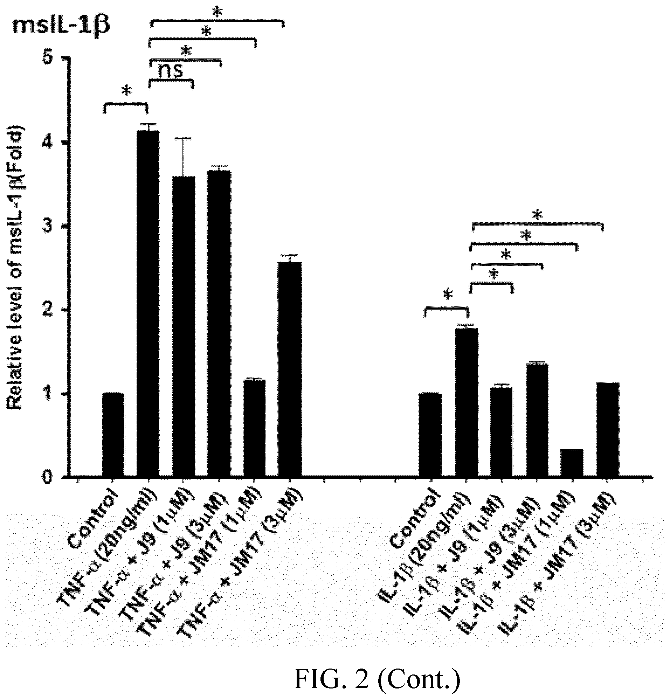

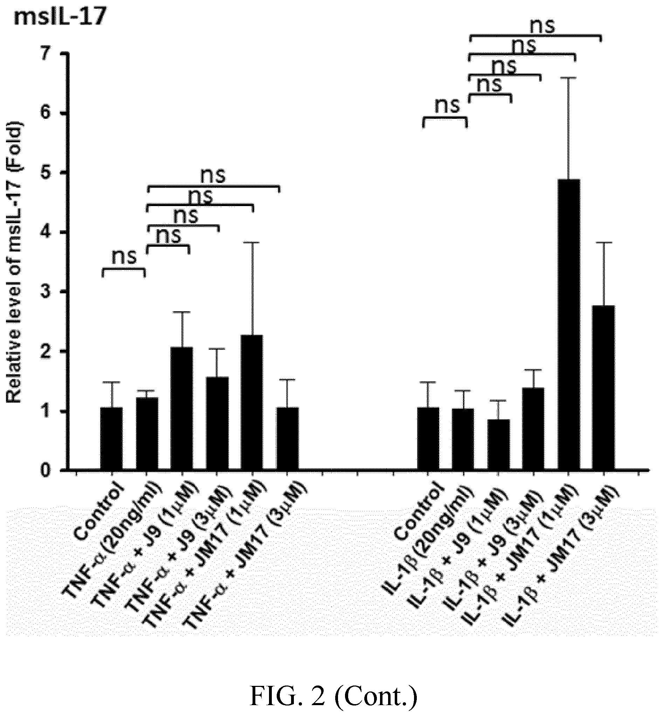

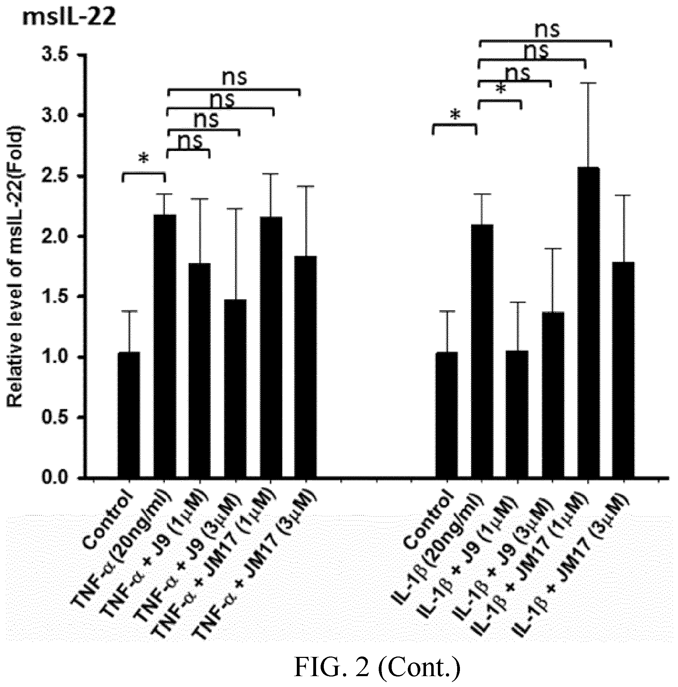

FIG. 2 shows effects of ASC-J9 (J9) and ASC-JM17 (JM17) on inflammatory cytokine induced cytokine expression in dendritic cells. Dendritic cells were pre-treated with different concentration of ASC-J9 and ASC-JM17 for 1 hour and then inflammatory cytokine TNF-α or IL-1β were added for 24 hours. Expression of mouse cytokine genes was measured by RT-qPCR. *: p < 0.05; ns: not significant.

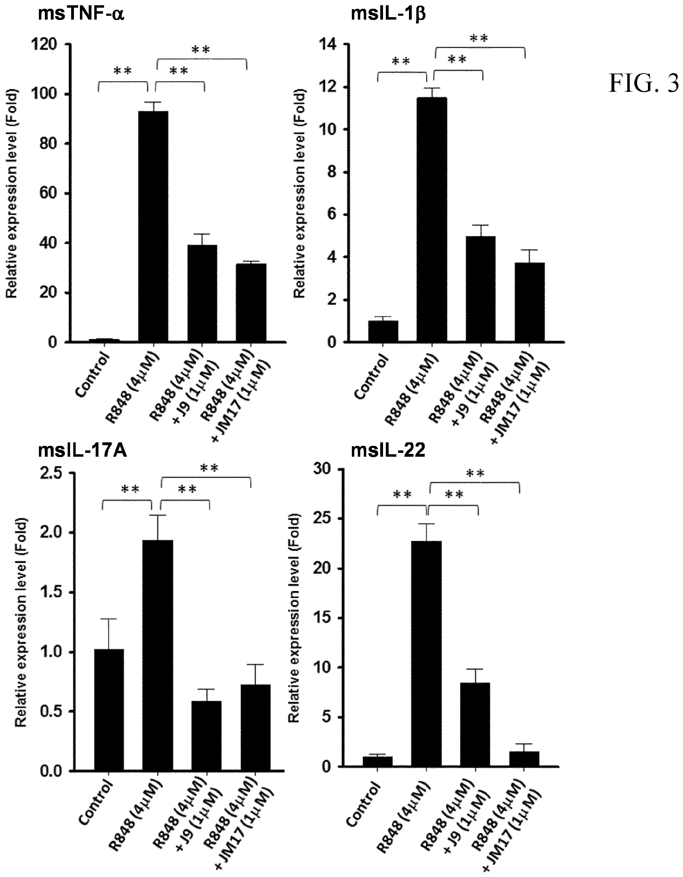

FIG. 3 shows effects of ASC-J9 (J9) and ASC-JM17 (JM17) on R848 induced cytokine expression in mouse splenocytes. Mouse splenocytes were pre-treated with 1 µM of ASC-J9 or ASC-JM17 for 1 hour and then TLR ligand, R848 were added for 6 hours. Expression of mouse cytokine genes were measured by RT-qPCR. *: p < 0.01.

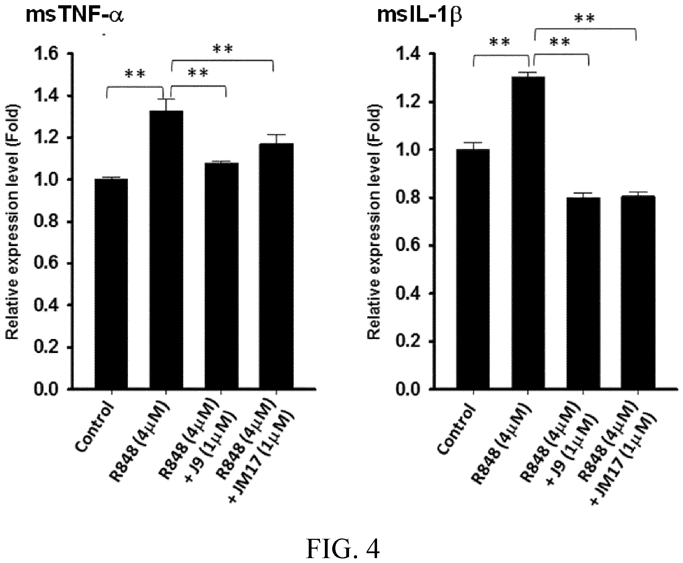

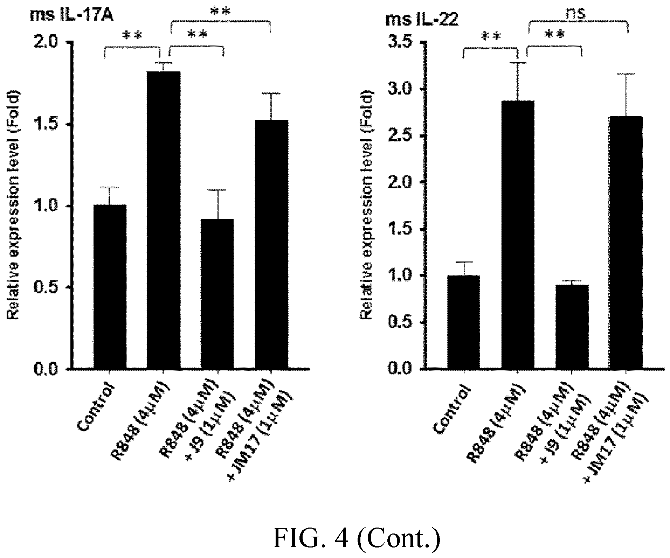

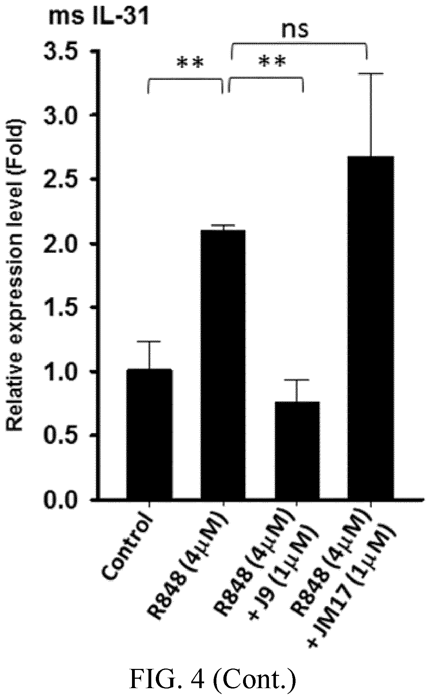

FIG. 4 shows effects of ASC-J9 (J9) and ASC-JM17 (JM17) on R848 induced cytokine expression in mouse bone marrow derived macrophages. Macrophages were pre-treated with 1 µM of ASC-J9 or ASC-JM17 for 1 hour and then TLR ligand, R848 were added for 6 hours. Expression of mouse cytokine genes were measured by RT-qPCR. *: p < 0.01; ns: not significant.

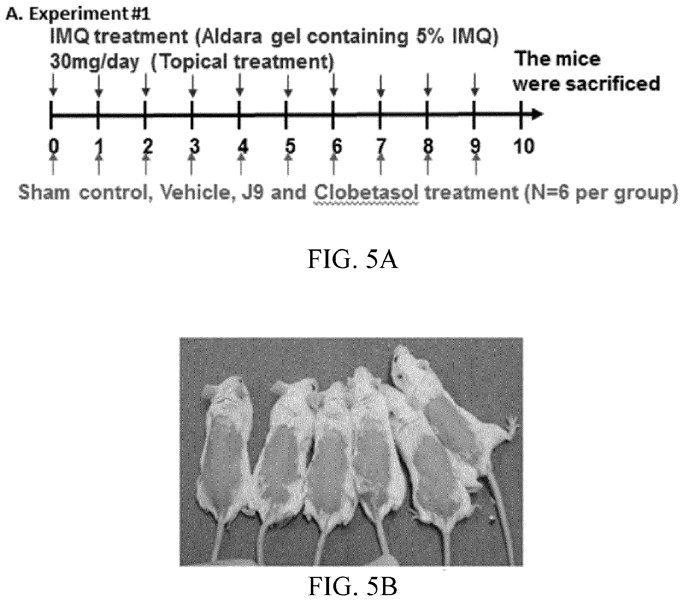

FIGs. 5A to 5D show evaluation of anti-inflammatory effects on ASC-J9 (J9) on IMQ-induced psoriatic inflammation *in vivo.* Balb/c mice were topical treated with imiquimod (IMQ) and with control cream, ASC-J9 or clobetasol for consecutive 10 days. (A) Schematic schedule was illustrated. Photographic observations in each group were shown at day 0 (B), day 5 (C) and day 10 (D).

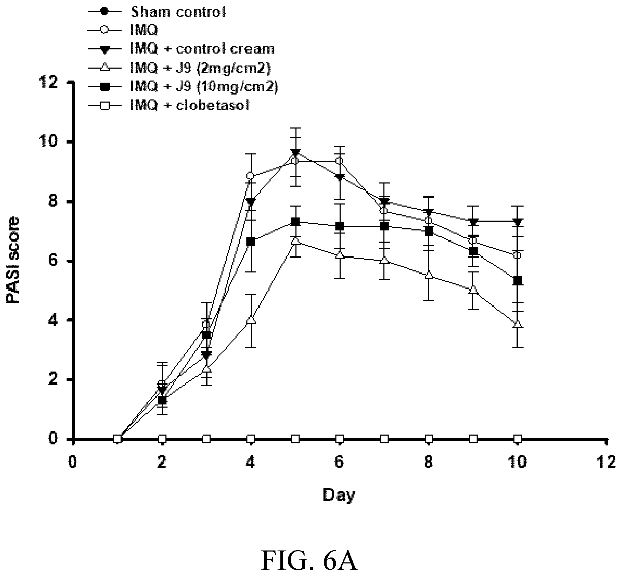

FIGs. 6A to 6D show evaluation of anti-inflammatory effects on ASC-J9 (J9) on IMQ-induced psoriatic inflammation *in vivo.* Balb/c mice were topical treated with imiquimod (IMQ) and with control cream, ASC-J9 or clobetasol for consecutive 10 days. (A) The severity of inflammatory responses on the skin was assessed on the basis of the Psoriasis Area Severity Index. (B) Ten days after treatment, the mice were sacrificed and their skin thickness was measured with vernier caliper to assess the severity of the psoriatic responses. (C) Expession of cytokine genes in mouse skin after 10 day treatment by RT-qPCR. (F) H&E staining of lesional skin after 10 day treatment. *: p < 0.05; **: p < 0.05; ns: not significant.

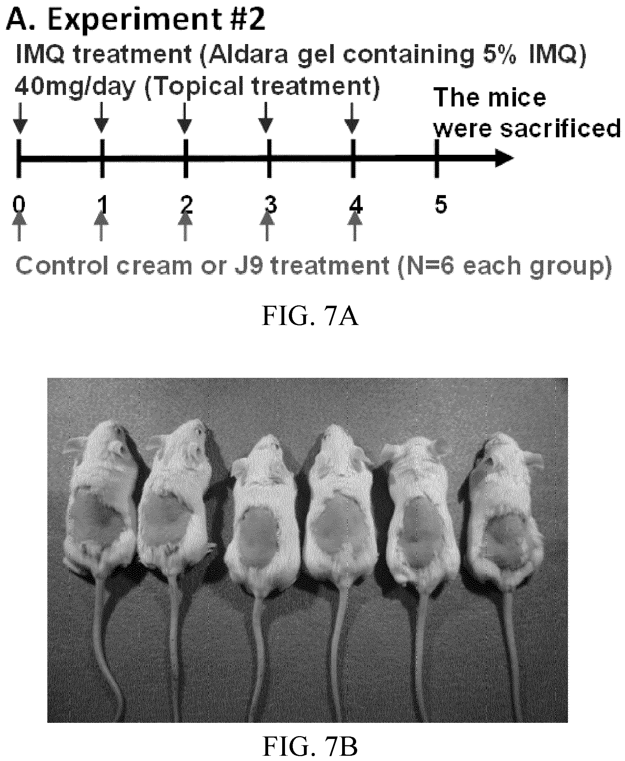

FIGs. 7A to 7C show evaluation of anti-inflammatory effects on ASC-J9 (J9) on IMQ-induced psoriatic inflammation *in vivo.* Balb/c mice were topical treated with imiquimod (IMQ) and with control cream, different dose of ASC-J9 for consecutive 5 days. (A) Schematic schedule was illustrated. Photographic observations in each group were shown at day 0 (B), day 5 (C).

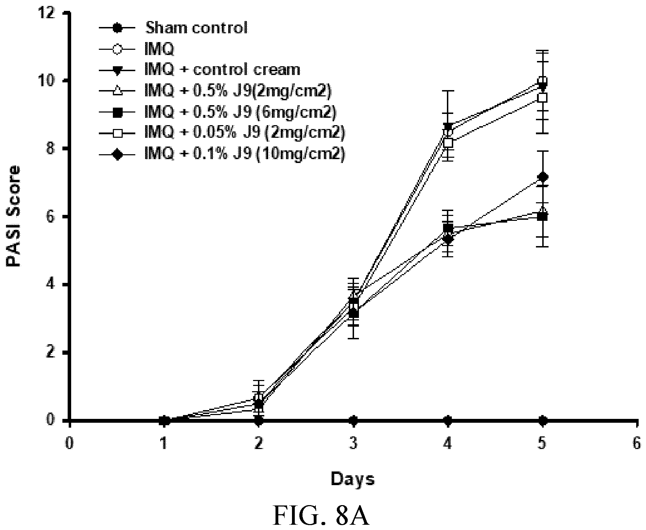

FIGs. 8A to 8C show evaluation of anti-inflammatory effects on ASC-J9 (J9) on IMQ-induced psoriatic inflammation *in vivo.* Balb/c mice were topical treated with imiquimod (IMQ) and with control cream, different dose of ASC-J9 for consecutive 5 days. (A) The severity of inflammatory responses on the skin was assessed on the basis of the Psoriasis Area Severity Index. (B) Five days after treatment, the mice were sacrificed and their skin thickness was measured with vernier caliper to assess the severity of the psoriatic responses. (C) Expession of cytokine genes in mouse skin after 10 day treatment by RT-qPCR. *: p < 0.05; **: p < 0.05; ns: not significant.

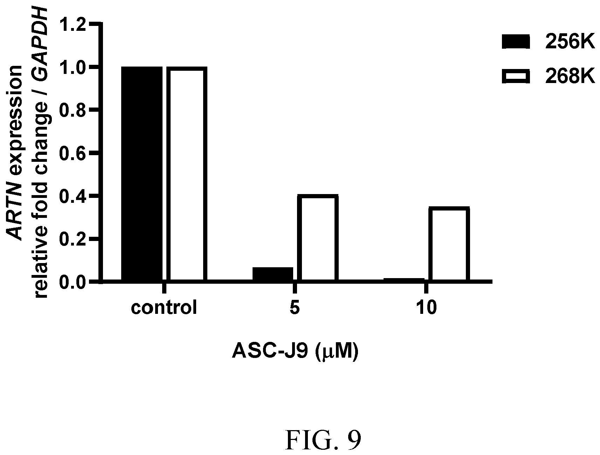

FIG. 9 shows ASC-J9 suppresses pruritus markers in keloid patient derived fibroblasts.

### DETAILED DESCRIPTION OF THE INVENTION

The present invention can be more readily understood by reference to the following detailed description of various embodiments of the invention, the examples, and the chemical drawings and tables with their relevant descriptions. It is to be understood that unless otherwise specifically indicated by the claims, the invention is not limited to specific preparation methods, carriers or formulations, or to particular modes of formulating the compound of the invention into products or compositions intended for topical, oral or parenteral administration, because as one of ordinary skill in the relevant arts is well aware, such things can, of course, vary. It is also to be understood that the terminology used herein is for the purpose of describing particular embodiments only and is not intended to be limiting.

The invention is to provide a method for treating an inflammatory disorder relating an inflammatory cytokine overexpression in a subject in need of such treatment, comprising administrating to said subject a pharmaceutical composition comprising an effective amount of a compound according to formula V or VIII and optionally a pharmaceutically acceptable carrier or excipient; 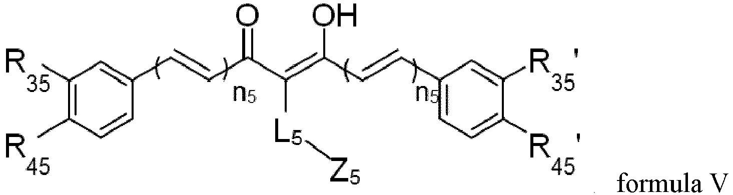 wherein in formula V:
each "n₅" is independently 1, 2, or 3;
R₃₅, R₄₅, R₃₅, and R₄₅ are independently selected from the group consisting of -H, - OH, and -OCH₃;
L₅-Z₅ is absent; or
L₅-Z₅ exists, and L₅ is selected from the group consisting of a C0-C8 alkylene, an unsaturated alkenylene, and alkynl when Z₅ is absent;
Z₅ is selected from the group consisting of -H, -OH, an aromatic ring, a cycloalkyl, -COR₁₅, -CO₂R₁₅, -CONR₁₅R₂₅, -NR₁₅R₂₅, -C(X₅)_{3;}
R₁₅ and R₂₅ are independently selected from the group consisting of -H, -CH₃, and -C₂H₅; and
X₅ is a halogen atom selected from the group consisting of -F, -Cl, and -Br; 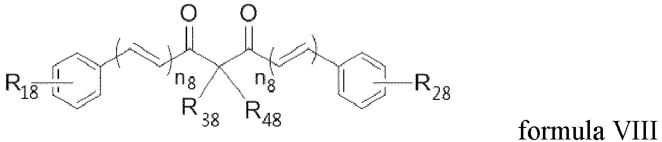
R₁₈ and R₂₈ are mono- or di-substituted groups and independently selected from the group consisting of a methoxy group, a hydroxyl group, and an alkyl sulfonyl group;
R₃₈ is selected from the group consisting of 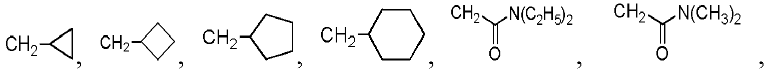 (CH₂)₃CH₃, 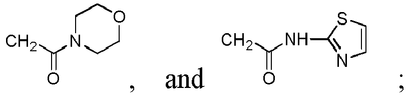 and
R₄₈ is selected from the group consisting of CH₃, H, F and Cl; and
n₈ is 1 or 2;
wherein the inflammatory cytokine is selected from the group consisting of INF-α, IL-1β, IL-17A, IL-22 and IL-31.

The term "(substituted phenyl)-propenal moiety" as used herein refers to a composition including a phenyl group having attached thereto a propenal moiety (when m equals 1) and an alkoxy or hydroxy moiety, or an alkyl or substituted alkyl moiety. The substitutions may be positioned meta or para or ortho with respect to the propenal moiety as used herein and refers to a general formula 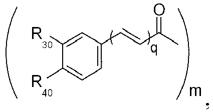 where q may be any number of 1, 2, 3 or 4; and m may be any number of 1, 2, 3, 4, or more.

The term "alkyl" as used herein refers to a straight or branched hydrocarbon chain radical consisting solely of carbon and hydrogen atoms, containing no unsaturation, having from one to ten carbon atoms, and which is attached to the rest of the molecule by a single bond, e.g. methyl, ethyl, n-propyl, 1-methylethyl (iso-propyl), n-butyl, n-pentyl, 1,1-dimethylethyl (t-butyl), and the like.

The term "alkenyl" as used herein refers to a straight or branched hydrocarbon chain radical consisting solely of carbon and hydrogen atoms, containing at least one double bond, having from two to ten carbon atoms, and which is attached to the rest of the molecule by a single bond or a double bond, e.g. ethenyl, prop-1-enyl, pent-1-enyl, penta-1,4-dienyl, and the like.

The term "alkenylene" as used herein refers to a straight or branched hydrocarbon chain which contains a carbon-to-carbon double bond and is represented by the formula CₚH₂ₚ₋₂, wherein hydrogen may be replaced by an additional carbon-to-carbon double bond or a monovalent substituent, e.g. ethenylene, prop-1-enylene and the like.

The term "alkoxy" as used herein refers to the radical having the formula -OR wherein R is an alkyl, haloalkyl or cycloalkyl. An "optionally substituted alkoxy" refers to the radical having the formula -OR' wherein R' is an optionally substituted alkyl as described herein.

The term "alkynl" as used herein refers to a straight or branched hydrocarbon chain radical consisting solely of carbon and hydrogen atoms, containing at least one triple bond, having from two to ten carbon atoms, and which is attached to the rest of the molecule by a single bond or a triple bond, e.g. ethynyl, prop-1-ynyl, but-1-ynyl, pent-1-ynyl, pent-3-ynyl and the like.

The term "aryl" as used herein refers to a radical of carbocyclic ring system wherein at least one of the rings is aromatic. The aryl may be fully aromatic or may contain an aromatic ring in combination with a non-aromatic ring. A "biaryl system" is a compound that includes at least two aryl groups.

The term "cycloalkyl" as used herein refers to a stable monovalent monocyclic or bicyclic hydrocarbon radical consisting solely of carbon and hydrogen atoms, having from three to ten carbon atoms, and which is saturated and attached to the rest of the molecule by a single bond, e.g. cyclopropyl, cyclobutyl, cyclopentyl, cyclohexyl, and the like.

The term "di-ketone bridge," or "ketone-enol bridge" as used herein, refers to a straight or branched hydrocarbon chain including two ketones or an enol positioned in close proximity to a ketone respectively. The "di-ketone bridge" or "ketone-enol bridge" is positioned between at least two aryl moieties.

The term "hydroxyalkyl" as used herein refers to a straight or branched hydroxy substituted hydrocarbon chain radical having from one to ten carbon atoms, e.g. -CH₂OH, -(CH₂)₂OH and the like.

In some embodiments the compound according to formula V is provided according to formula V-1 or V-2:

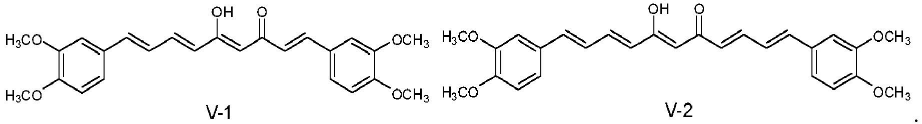

In one preferred embodiment of the invention, the compounds according to formula VIII including 4,4-disubstituted 1,7-bis-(3,4-dimethoxyphenyl)-hepta-1,6-diene-3,5-dione and 6,6-disubstituted 1,11-bis(substitutedphenyl)-undeca-1,3,8,10-tetraene-5,7-dione scaffolds are as shown in Table 1.

**Table 1:**

| | | | | | | |
|---|---|---|---|---|---|---|
| 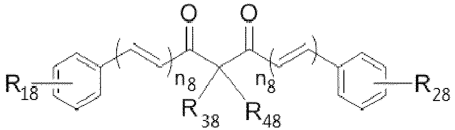 | | | | | | |

| **Compound ID** | **R₁₈** | **R₂₈** | **R₃₈** | **R₄₈** | **n₈** | **formula** |
|---|---|---|---|---|---|---|
| 1 | 3'4'-OCH₃ | 3'4'-OCH₃ | 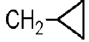 | CH₃ | 1 | C₂₈H₃₂O₆ |
| | | | | | | 464.55 |
| 2 | 3'4'-OCH₃ | 3'4'-OCH₃ | 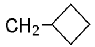 | CH₃ | 1 | C₂₉H₃₄O₆ |
| | | | | | | 478.5767 |
| 3 | 3'4'-OCH₃ | 3'4'-OCH₃ | 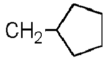 | H | 1 | C₂₉H₃₄O₆ |
| | | | | | | 478.58 |
| 4 | 3'4'-OCH₃ | 3'4'-OCH₃ | 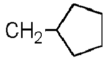 | CH₃ | 1 | C₃₀H₃₆O₆ |
| | | | | | | 492.60 |
| 5 | 3'4'-OCH₃ | 3'4'-OCH₃ | 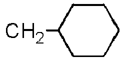 | CH₃ | 1 | C₃₁H₃₈O₆ |
| | | | | | | 506.63 |
| 6 | 3'4'-OCH₃ | 3'4'-OCH₃ | 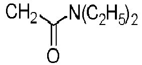 | CH₃ | 1 | C₃₀H₃₇NO₇ |
| | | | | | | 523.62 |
| 7 | 3'4'-OCH₃ | 3'4'-OCH₃ | 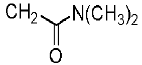 | CH₃ | 1 | C₂₈H₃₃NO₇ |
| | | | | | | 495.56 |
| 8 | 3'4'-OCH₃ | 3'4'-OCH₃ | (CH₂)₃CH₃ | CH₃ | 1 | C₂₈H₃₄O₆ |
| | | | | | | 466.57 |
| 9 | 3'4'-OCH₃ | 3'4'-OCH₃ | 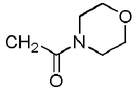 | H | 1 | C₂₉H₃₃NO₈ |
| | | | | | | 523.57 |
| 10 | 3'4'-OCH₃ | 3'4'-OCH₃ | 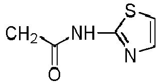 | H | 1 | C₂₈H₂₈N₂O₇S |
| | | | | | | 536.60 |
| 11 | 3'4'-CH₃ | 3'4'-CH₃ | 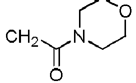 | CH₃ | 1 | C₃₀H₃₅NO₈ |
| | | | | | | 537.60 |
| 12 | 3'4'-OCH₃ | 3'4'-OCH₃ | 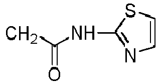 | CH₃ | 1 | C₂₉H₃₀N₂O₇S |
| | | | | | | 550.62 |
| 13 | 3'4'-OCH₃ | 3'4'-OCH₃ | 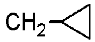 | F | 1 | C₂₇H₂₉FO₆ |
| | | | | | | 468.51 |
| 14 | 3'4'-OCH₃ | 3'4'-OCH₃ | 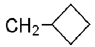 | F | 1 | C₂₈H₃₁FO₆ |
| | | | | | | 482.54 |
| 15 | 3'4'-OCH₃ | 3'4'-OCH₃ | 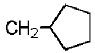 | F | 1 | C₂₉H₃₃FO₆ |
| | | | | | | 496.57 |
| 16 | 3'4'-OCH₃ | 3'4'-OCH₃ | 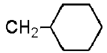 | F | 1 | C₃₀H₃₅FO₆ |
| | | | | | | 510.59 |
| 17 | 3'4'-OCH₃ | 3'4'-OCH₃ | 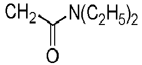 | F | 1 | C₂₉H₃₄FNO₇ |
| | | | | | | 527.58 |
| 18 | 3'4'-OCH₃ | 3'4'-OCH₃ | 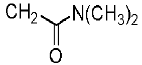 | F | 1 | C₂₇H₃₀FNO₇ |
| | | | | | | 499.53 |
| 19 | 3'4'-OCH₃ | 3'4'-OCH₃ | (CH₂)₃CH₃ | F | 1 | C₂₇H₃₁FO₆ |
| | | | | | | 470.53 |
| 20 | 3'4'-OCH₃ | 3'4'-OCH₃ | 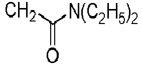 | Cl | 1 | C₂₉H₃₄ClNO₇ |
| | | | | | | 544.04 |
| 21 | 3'4'-OCH₃ | 3'4'-OCH₃ | 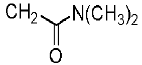 | Cl | 1 | C₂₇H₃₀ClNO₇ |
| | | | | | | 515.98 |
| 22 | 3'4'-OCH₃ | 3'4'-OCH₃ | CH₂CH₂COOC₂H₅ | CH₃ | 1 | C₂₉H₃₄O₈ |
| | | | | | | 510.58 |
| 23 | 3'4'-OCH₃ | 3'4'-OCH₃ | CH₂CH₂COOC₂H₅ | F | 1 | C₂₈H₃₁FO₈ |
| | | | | | | 514.54 |
| 24 | 3'4'-OCH₃ | 3'4'-OCH₃ | CH₂CH₂COOH | CH₃ | 1 | C₂₇H₃₀O₈ |
| | | | | | | 482.52 |
| 25 | 3'4'-OCH₃ | 3'4'-OCH₃ | CH₂CH₂COOH | F | 1 | C₂₆H₂₇FO₈ |
| | | | | | | 486.49 |
| 26 | 3'OCH_{3,}4'OH | 3'OCH_{3,}4'OH | CH₂CH₂COOC₂H₅ | CH₃ | 1 | C₂₇H₃₀O₈ |
| | | | | | | 482.52 |
| 27 | 3'OCH_{3,}4'OH | 3'OCH_{3,}4'OH | CH₂CH₂COOC₂H₅ | F | 1 | C₂₆H₂₇FO₈ |
| | | | | | | 486.49 |
| 28 | 3'-OCH₃ | 3'-OCH₃ | 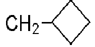 | CH₃ | 1 | C₂₇H₃₀O₄ |
| | | | | | | 418.52 |
| 29 | 3'-OH | 3'-OH | 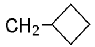 | CH₃ | 1 | C₂₅H₂₆O₄ |
| | | | | | | 390.47 |
| 30 | 3'-OCH₃ | 3'-OSO₂C₂H₅ | 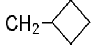 | CH₃ | 1 | C₂₈H₃₂O₆S |
| | | | | | | 496.62 |
| 31 | 3'-OSO₂C₂H₅ | 3'-OSO₂C₂H₅ | 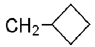 | CH₃ | 1 | C₂₉H₃₄O₈S₂ |
| | | | | | | 574.71 |
| 32 | 3'4'-OCH₃ | 3'4'-OCH₃ | 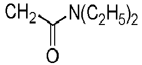 | CH₃ | 2 | C₃₄H₄₁NO₇ |
| | | | | | | 575.69 |
| 33 | 3'4'-OCH₃ | 3'4'-OCH₃ | 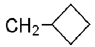 | F | 2 | C₃₂H₃₅FO₆ |
| | | | | | | 534.62 |
| 34 | 3'4'-OCH₃ | 3'4'-OCH₃ | 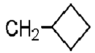 | H | 1 | C₂₈H₃₂O₆ |
| | | | | | | 464.55 |

In another preferred embodiment of the invention, the compound is formula V, and "n₅" is 1; R₃₅, R₄₅, R₃₅, and R₄₅ are -OCH₃; and L₅-Z₅ side chain is absent, referred as ASC-J9.

In another preferred embodiment of the invention, the compound is formula VIII, R₁₈ and R₂₈ are di-substituted methoxy groups, R₃₈ is 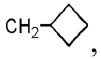 R₄₈ is H, and n₈ is 1, referred as ASC-JM17.

The invention also provides a composition comprising the compound with (substituted phenyl)-propenal moiety. The composition according to the invention is preferably a pharmaceutical composition, food composition or a cosmetic composition.

Preferably, the pharmaceutical composition comprises the compound with (substituted phenyl)-propenal moiety and optionally a pharmaceutically acceptable carrier or excipient.

Preferably, the pharmaceutical composition comprises an effective amount of the compound.

Often, ranges are expressed herein as from "about" one particular value and/or to "about" another particular value. When such a range is expressed, an embodiment includes the range from the one particular value and/or to the other particular value. Similarly, when values are expressed as approximations, by use of the word "about," it will be understood that the particular value forms another embodiment. It will be further understood that the endpoints of each of the ranges are significant both in relation to and independently of the other endpoint.

"Optional" or "optionally" means that the subsequently described event or circumstance may or may not occur, and that the description includes instances where said event or circumstance occurs and instances where it does not. For example, the phrase "optionally comprising an agent" means that the agent may or may not exist.

It must be noted that, as used in the specification and the appended claims, the singular forms "a," "an" and "the" include plural referents unless the context clearly dictates otherwise. Thus, unless otherwise required by context, singular terms shall include the plural and plural terms shall include the singular.

The term "subject" as used herein denotes any animal, preferably a mammal, and more preferably a human. The examples of subjects include humans, non-human primates, rodents, guinea pigs, rabbits, sheep, pigs, goats, cows, horses, dogs and cats.

The term "effective amount" of an active ingredient as provided herein means a sufficient amount of the ingredient to provide the desired regulation of a desired function. As will be pointed out below, the exact amount required will vary from subject to subject, depending on the disease state, physical conditions, age, sex, species and weight of the subject, the specific identity and formulation of the composition, etc. Dosage regimens may be adjusted to induce the optimum therapeutic response. For example, several divided doses may be administered daily or the dose may be proportionally reduced as indicated by the exigencies of the therapeutic situation. Thus, it is not possible to specify an exact "effective amount." However, an appropriate effective amount can be determined by one of ordinary skill in the art using only routine experimentation.

The term "treating" or "treatment" as used herein denotes reversing, alleviating, inhibiting the progress of, or improving the disorder, disease or condition to which such term applies, or one or more symptoms of such disorder, disease or condition.

The term "carrier" or "excipient" as used herein refers to any substance, not itself a therapeutic agent, used as a carrier and/or diluent and/or adjuvant, or vehicle for delivery of a therapeutic agent to a subject or added to a formulation to improve its handling or storage properties or to permit or facilitate formation of a dose unit of the composition into a discrete article such as a capsule or tablet suitable for oral administration. Suitable carriers or excipients are well known to persons of ordinary skill in the art of manufacturing pharmaceutical formulations or food products. Carriers or excipients can include, by way of illustration and not limitation, buffers, diluents, disintegrants, binding agents, adhesives, wetting agents, polymers, lubricants, glidants, substances added to mask or counteract a disagreeable taste or odor, flavors, dyes, fragrances, and substances added to improve appearance of the composition. Acceptable carriers or excipients include citrate buffer, phosphate buffer, acetate buffer, bicarbonate buffer, stearic acid, magnesium stearate, magnesium oxide, sodium and calcium salts of phosphoric and sulfuric acids, magnesium carbonate, talc, gelatin, acacia gum, sodium alginate, pectin, dextrin, mannitol, sorbitol, lactose, sucrose, starches, gelatin, cellulosic materials (such as cellulose esters of alkanoic acids and cellulose alkyl esters), low melting wax cocoa butter, amino acids, urea, alcohols, ascorbic acid, phospholipids, proteins (for example, serum albumin), ethylenediamine tetraacetic acid (EDTA), dimethyl sulfoxide (DMSO), sodium chloride or other salts, liposomes, mannitol, sorbitol, glycerol or powder, polymers (such as polyvinylpyrrolidone, polyvinyl alcohol, and polyethylene glycols), and other pharmaceutically acceptable materials. The carrier should not destroy the pharmacological activity of the therapeutic agent and should be non-toxic when administered in doses sufficient to deliver a therapeutic amount of the agent.

The pharmaceutical composition according to the invention is preferably administered topically or systemically by any method known in the art, including, but not limited to, intramuscular, intradermal, intravenous, subcutaneous, intraperitoneal, intranasal, oral, mucosal or external routes. The appropriate route, formulation and administration schedule can be determined by those skilled in the art. In the present invention, the pharmaceutical composition can be formulated in various ways, according to the corresponding route of administration, such as a liquid solution, a suspension, an emulsion, a syrup, a tablet, a pill, a capsule, a sustained release formulation, a powder, a granule, an ampoule, an injection, an infusion, a kit, an ointment, a lotion, a liniment, a cream or a combination thereof. If necessary, it may be sterilized or mixed with any pharmaceutically acceptable carrier or excipient, many of which are known to one of ordinary skill in the art.

The external route as used herein is also known as local administration, includes but is not limited to administration by insufflation and inhalation. Examples of various types of preparation for local administration include ointments, lotions, creams, gels, foams, preparations for delivery by transdermal patches, powders, sprays, aerosols, capsules or cartridges for use in an inhaler or insufflator or drops (e.g. eye or nose drops), solutions/suspensions for nebulisation, suppositories, pessaries, retention enemas and chewable or suckable tablets or pellets or liposome or microencapsulation preparations.

Ointments, creams and gels, may, for example, be formulated with an aqueous or oily base with the addition of suitable thickening and/or gelling agent and/or solvents. Such bases may thus, for example, include water and/or oil such as liquid paraffin or a vegetable oil such as arachis oil or castor oil, or a solvent such as polyethylene glycol. Thickening agents and gelling agents which may be used according to the nature of the base include soft paraffin, aluminium stearate, cetostearyl alcohol, polyethylene glycols, woolfat, beeswax, carboxypolymethylene and cellulose derivatives, and/or glyceryl monostearate and/or non-ionic emulsifying agents.

Lotions may be formulated with an aqueous or oily base and will in general also contain one or more emulsifying agents, stabilising agents, dispersing agents, suspending agents or thickening agents.

Powders for external application may be formed with the aid of any suitable powder base, for example, talc, lactose or starch. Drops may be formulated with an aqueous or non-aqueous base also comprising one or more dispersing agents, solubilising agents, suspending agents or preservatives.

Spray compositions may for example be formulated as aqueous solutions or suspensions or as aerosols delivered from pressurised packs, such as a metered dose inhaler, with the use of a suitable liquefied propellant. Aerosol compositions suitable for inhalation can be either a suspension or a solution. The aerosol composition may optionally contain additional formulation excipients well known in the art such as surfactants e.g. oleic acid or lecithin and cosolvents e.g. ethanol.

Topical preparations may be administered by one or more applications per day to the affected area; over the skin areas occlusive dressings may advantageously be used. Continuous or prolonged delivery may be achieved by an adhesive reservoir system.

The cosmetic composition according to the invention may be an aqueous phase formulation consisting essentially of water; it may also comprise a mixture of water and of water-miscible solvent (miscibility in water of greater than 50% by weight at 25°C), for instance lower monoalcohols containing from 1 to 5 carbon atoms such as ethanol or isopropanol, glycols containing from 2 to 8 carbon atoms, such as propylene glycol, ethylene glycol, 1,3-butylene glycol or dipropylene glycol, C3-C4 ketones and C2-C4 aldehydes, and glycerin. Such an aqueous formulation preferably is in a form of aqueous gel or hydrogel formulation. The hydrogel formulation comprises a thickening agent to thicken the liquid solution. Examples of the thickening agents include, but are not limited to, carbomers, cellulose base materials, gums, algin, agar, pectins, carrageenan, gelatin, mineral or modified mineral thickeners, polyethylene glycol and polyalcohols, polyacrylamide and other polymeric thickeners. The thickening agents which give the stability and optimal flow characteristics of the composition are preferably used.

The cosmetic composition according to the present invention may be in a form of emulsion or cream formulation. It can contain emulsifying surfactants. These surfactants may be chosen from anionic and nonionic surfactants. Reference may be made to the document "Encyclopedia of Chemical Technology, Kirk-Othmer", volume 22, pp. 333-432, 3rd edition, 1979, Wiley, for the definition of the properties and functions (emulsifying) of surfactants, in particular pp. 347-377 of said reference, for the anionic and nonionic surfactants.

The surfactants preferably used in the cosmetic composition according to the invention are chosen from: nonionic surfactants: fatty acids, fatty alcohols, polyethoxylated or polyglycerolated fatty alcohols such as polyethoxylated stearyl or cetylstearyl alcohol, fatty acid esters of sucrose, alkylglucose esters, in particular polyoxyethylenated fatty esters of C1-C6 alkyl glucose, and mixtures thereof; anionic surfactants: C16-C30 fatty acids neutralized with amines, aqueous ammonia or alkaline salts, and mixtures thereof. Surfactants which make it possible to obtain an oil-in-water or wax-in-water emulsion are preferably used.

The cosmetic composition according to the invention may further comprise an effective amount of a physiologically acceptable antioxidant selected from the group consisting of butylated p-cresol, butylated hydroquinone monomethyl ether, and a tocopherol.

The cosmetic composition according to the invention may further comprise natural or modified amino acid, natural or modified sterol compound, natural or modified collagen, silk protein or soy protein.

The cosmetic composition according to the invention is preferably formulated for topical application to keratin materials such as the skin, the hair, the eyelashes or the nails. They may be in any presentation form normally used for this type of application, especially in the form of an aqueous or oily solution, an oil-in-water or water-in-oil emulsion, a silicone emulsion, a microemulsion or nanoemulsion, an aqueous or oily gel or a liquid, pasty or solid anhydrous product.

The cosmetic composition according to the invention may be more or less fluid and may have the appearance of a white or colored cream, an ointment, a milk, a lotion, a serum, a paste, a mousse or a gel. It may optionally be topically applied onto the skin in the form of an aerosol, a patch or a powder. It may also be in solid form, for example, in the form of a stick. It may be used as care products and/or as makeup products for the skin. Alternatively, it may be formulated as shampoos or conditioners.

In known fashion, the cosmetic composition according to the invention may also contain additives and adjuvants that are common in cosmetics, such as hydrophilic or lipophilic gelling agents, preservatives, antioxidants, solvents, fragrances, fillers, pigments, odor absorbers and dyestuffs.

The compound can be added to a conventional food composition (i.e. the edible food or drink or precursors thereof) in the manufacturing process of the food composition. Almost all food compositions can be supplemented with the compound of the invention. The food compositions that can be supplemented with the compound of the invention include, but are not limited to, candies, baked goods, ice creams, dairy products, sweet and flavor snacks, snack bars, meal replacement products, fast foods, soups, pastas, noodles, canned foods, frozen foods, dried foods, refrigerated foods, oils and fats, baby foods, or soft foods painted on breads, or mixtures thereof.

In a preferred embodiment of the invention, the pharmaceutical composition is a topical pharmaceutical composition.

In one preferred embodiment of the invention, the pharmaceutical composition further comprises an emollient, skin penetrant, emulsifying agent, neutralizing agent, gelling agent, preservative, or chelating agent.

The invention also provides a method for treating an inflammatory disorder relating an inflammatory cytokine overexpression in a subject in need of such treatment, comprising administrating to said subject the pharmaceutical composition as mentioned above.

While not wishing to be limited by theory, it is Applicant's belief that the method according to the invention is treating the disorder through suppressing inflammatory cytokine synthesized by activated dendritic cells, macrophages, or T-cells.

Preferably, the inflammatory cytokine contains but is not limited to the INF-α, IL-1β, IL-17A, IL-22 and IL-31.

In one preferred embodiment of the invention, the disorder is an epidermal disorder.

In another preferred embodiment of the invention, the disorder is a dermal disorder.

In another aspect, the disorder is preferably an inflammatory skin disorder.

Preferably, the inflammatory skin disorder contains but is not limited to actinic keratosis, atopic dermatitis, chickenpox, cold sore, contact dermatitis, eczema, hives, impetigo, keratosis pilaris, latex allergy, measles, psoriasis, ringworm, rosacea, keloids, hypertrophic scars, pruritus, or seborrheic eczema.

The invention also provides a method for treating an autoimmune disease in a subject in need of such treatment, comprising administrating to said subject the pharmaceutical composition as mentioned above.

In one preferred embodiment of the invention, the autoimmune disease contains but is not limited to Addison's disease, celiac disease, graft versus host disease (GvHD), Graves' disease, Hashimoto's thyroiditis, inflammatory bowel diseases, multiple sclerosis, myasthenia gravis, pernicious anemia, psoriatic arthritis, rheumatoid arthritis (RA), Sjögren's syndrome, systemic lupus erythematosus (lupus), type 1 diabetes, or vasculitis.

Preferably, the inflammatory bowel diseases contain but are not limited to ulcerative colitis or Crohn's disease.

In one embodiment of the invention, we have established the imiquimod (IMQ)-induced psoriatic model in which imiquimod induces psoriatic inflammation on mouse skin that resembles to the human psoriatic response. We used immune cells including dendritic cells and macrophage as well as this IMQ-induced psoriatic mouse model to evaluate the effect of ASC-J9 and related compounds on treatment of psoriasis inflammation. In *in vitro* studies, we found that ASC-J9 and ASC-JM17 could inhibit inflammatory cytokine- or TLR ligand-induced cytokine expression in mouse splenocytes, mouse bone marrow derived dendritic cells and bone marrow derived macrophages. In addition, we also observed that ASC-J9 cream attenuated IMQ-induced psoriatic inflammation *in vivo.* In addition, J9 attenuates IMQ-induced psoriatic inflammation animal model, which includes reducing erythema, decreasing scaling and thickness of lesional skin, and inhibition of inflammatory gene expression in the psoriatic skin. Taken these results together, ASC-J9 and ASC-JM17 exert anti-inflammatory activities to inhibit psoriatic inflammation.

In one preferred embodiment of the invention, ASC-J9 suppresses pruritus markers in keloid patient derived fibroblasts. While not wishing to be limited by theory, it is Applicant's belief that ASC-J9 suppresses pruritus through down-regulating artemin (ARTN) expression and transient receptor potential (TRP) channels in patient derived keloid fibroblasts. Overexpression of ARTN is shown to enhance expression of TRPV1 and TRPA1 in cutaneous sensory neurons leading to pruritus (Mol Pain. 2015 Mar 8;11:8; Pharmaceuticals (Basel). 2018 Dec; 11(4): 100).

The following examples are provided to aid those skilled in the art in practicing the present invention.

### EXAMPLES

### Example 1: Psoriasis

### Materials and Methods

### Storage Conditions of test articles

ASC-J9 (J9) and ASC-JM17 (JM17) powder for in vitro study were dissolved in DMSO and were stored at -20°C.

J9 control cream and J9 cream including 0.05% J9, 0.1% J9 and 0.5% J9 were stored at room temperature.

### Dose Preparation

J9 and JM17 powder were dissolved into 10 mg/mL in DMSO as stock and then serial diluted into various concentration for study.

### Test System:

MTS assay for cell viability: The cell toxicity of test articles is measured by MTS assay. The MTS assay is based on the reduction of the MTS tetrazolium compound by viable cells to generate a colored formazan dye that is soluble in cell culture media. This conversion is thought to be carried out by NAD(P)H-dependent dehydrogenase enzymes in metabolically active cells. The formazan dye is quantified by measuring the absorbance at 490-500 nm. The non-toxic dose will be applied for further assays.

Cell culture: Mouse bone marrow derived dendritic cells (BMDC) and bone marrow derived macrophages (BMDM) were generated from mouse bone marrow cells. Briefly, dendritic cells can be generated by two induction procedures. Bone marrow cells can be induced into myeloid DCs called GMDC with GM-CSF and IL-4 for 10 days, while a mixed types of DCs including myeloid and plasmacytoid DCs called Fit3DC can be generated by induction with fit3-ligand for 10 days. To generate BMDMs, bone marrow cells are induced into BMDM by 30% L929 conditioned medium for 7 days.

Analysis of gene expression: Total RNA was purified from cells using Isol-RNA Lysis Reagent (5 PRIME GmbH, Hilden, Germany) according to the manufacturer's protocol. Reverse-transcription was performed using the QuantiNova® Reverse Transcription Kit (QIAGEN™) and oligo-dT primers for first-strand cDNA synthesis. Quantitative PCR was performed with gene-specific primers (see appendix) using LightCycler480 detection system (Roche Diagnostics GmbH, Penzberg, Germany) and QuantiNova® SYBR Green RT-PCR Kit for gene expression analysis. The level of mRNA expression was normalized to that of GAPDH.

Animal Model of Psoriatic Inflammation: 30 or 40 mg of 5% IMQ gel (Aldara™) was smeared on the shaved backs of Balb/c mice each day for 5 or 10 days. The severity of the skin's inflammatory response was assessed on the basis of the Psoriasis Area Severity Index (PASI) as described. Briefly, the three parameters of psoriasis responses-erythema, scaling, and skin thickness-were scored independently on a scale from 0 to 4 as follows: 0: none; 1: slight; 2: moderate; 3: marked; and 4: very marked. By adding the scores from these three parameters, the severity of the response was measured using the cumulative score from 0 to 12. At the end of the experiment, the mice were sacrificed for measurement of skin thickness with vernier caliper. Group Assignments and Dose Levels of animal studies are described as follows: There are two animal experiments listed in Tables 2 and 3:

**Table 2: #1 Animal experiment:**

| **Group No.** | **Number of Animals (N)** | **Test Article** | **Test Article Dose (mg/kg)** | **Route** | **Frequency** |
|---|---|---|---|---|---|
| 1 | 5 | Sham control | 0 | none | none |
| 2 | 5 | IMQ (Aldara gel) | IMQ (Aldara gel) 30mg | Topical treatment | daily |
| 3 | 5 | IMQ (Aldara gel)+ J9 control cream | IMQ 30mg + J9 control cream 10mg/cm² | Topical treatment | daily |
| 4 | 5 | IMQ (Aldara gel)+ J9 (0.5% 2mg/cm²) | IMQ 30mg+ J9 2mg/cm² | Topical treatment | daily |
| 5 | 5 | IMQ (Aldara gel)+ J9 (0.5% 10mg/cm²) | IMQ 30mg+ J9 10mg/cm² | Topical treatment | daily |
| 6 | 5 | IMQ (Aldara gel)+ clobetasol (0.05% 8mg/cm²) | IMQ 30mg+ clobetasol 8mg/cm² | Topical treatment | daily |

**Table 3: #2 animal experiment:**

| **Group No.** | **Number of Animals (N)** | **Test Article** | **Test Article Dose (mg/kg)** | **Route** | **Frequency** |
|---|---|---|---|---|---|
| 1 | 5 | Sham control | none | none | none |
| 2 | 5 | IMQ (Aldara gel) | IMQ 40mg | Topical treatment | daily |
| 3 | 5 | IMQ (Aldara gel)+ J9 control cream | IMQ 40mg + J9 control cream 10mg/cm² | Topical treatment | daily |
| 4 | 5 | IMQ (Aldara gel) + J9 (0.5% 2mg/cm²) | IMQ 40mg+ J9 2mg/cm² | Topical treatment | daily |
| 5 | 5 | IMQ (Aldara gel)+ J9 (0.5% 6mg/cm²) | IMQ 40mg+ J9 10mg/cm² | Topical treatment | daily |
| 6 | 5 | IMQ (Aldara gel)+ J9 (0.1% 10mg/cm²) | IMQ 40mg+ J9 10mg/cm² | Topical treatment | daily |
| 7 | 5 | IMQ (Aldara gel)+ J9 (0.05% 10mg/cm²) | IMQ 40mg+ J9 10mg/cm² | Topical treatment | daily |

Statistical Analyses: All data are presented as the means ± SD. Statistical analyses were performed on the data from three independent experiments using Student's t-test. A P value < 0.05 was considered to be a statistically significant difference among the experimental groups.

### Data Analysis

### In vitro anti-inflammatory activity of J9 and JM17 on macrophages and dendritic cells

*In vitro* toxicity test was performed by MTS assay.

Anti-inflammatory effects of J9 and JM17 for inflammatory cytokine including TNF-α, IL-1β, IL-17A, IL-22, IL-23 and IL-31 induced by TLR ligand in various immune cells including mouse splenocytes, mouse bone marrow derived dendritic cells and bone marrow derived macrophages were analyzed by quantitative PCR (RT-qPCR).

The inhibitory effect of J9 and JM17 on animal model with imiquimod (IMQ)-induced psoriatic inflammation.

The mice were (1) photoed for evaluation and analyzed (2) Psoriasis Area Severity Index (PASI); (3) Skin thickness measurement. We selected the sample in experiment with better inhibitory effect for further analysis by accessing: (1) H&E staining and (2) inflammatory gene expression including TNF-α, IL-1β, IL-17A, IL-22, IL-23 and IL-31 in lesional tissue by quantitative PCR (RT-qPCR).

### Results

### In vitro anti-inflammatory activity of J9 and JM17 on macrophages and dendritic cells

The goal of this example is to evaluate the anti-inflammatory abilities of J9 and JM17 on psoriatic inflammation *in vitro* and *in vivo.* To find the safety doses of J9 and JM17 for in vitro study, cytotoxicity of J9 and JM17 were measured by MTS assay. Given that dendritic cells and macrophages are important cells to initiate psoriatic inflammation, these two cells were chosen for cytotoxicity test. These cells were treated with different concentrations of J9 and JM17 from 0 µM to 10 µM. As shown in FIG. 1, GMDC, Fit3DC and BMDM were resistant to J9 (non-toxic doses) up to 10 µM, 10 µM and 1 µM, respectively. In addition, GMDC, Fit3DC and BMDM were resistant to JM17 (non-toxic doses) up to 5 µM, 3 µM and 1 µM, respectively.

To evaluate the anti-inflammatory effects of J9 and JM17 *in vitro,* GMDC were pre-treated with J9 and JM17 for 1hr and stimulated with the inflammatory cytokine, TNF-α or IL-1β • for 24 hrs. Expression of cytokines including TNF-α, IL-1β, IL-17A, IL-22, IL-23, and IL-31 were measured by RT-qPCR. In FIG. 2, J9 and JM17 significantly inhibit TNF-α or IL-1β induced TNF-α and IL-1β expression in GMDC but J9 and JM17 did not attenuate TNF-α or IL-1β induced IL-22 expression in GMDC. Expression of IL-17A was not induced by TNF-α or IL-1β. Moreover, the expression of IL-23 and IL-31 were undetectable in dendritic cells among experimental groups.

We also evaluate anti-inflammatory effects of J9 and JM17 on mouse splenocytes and bone marrow derived macrophages. Mouse splenocytes were pre-treated with J9 and JM17 for 1hr and TLR ligand, R848, were then added for 6 hrs. RT-qPCR analysis revealed that J9 and JM17 significantly inhibited R848 induced TNF-α, IL-1β, IL-17A and IL-22 expression in mouse splenocytes, but expression of IL-23 and IL-31 were undetectable in mouse splenocytes (FIG. 3). In BMDM, we found that R848-induced expression of TNF-α, IL-1β, IL-17A, IL-22 and IL-31 were inhibited by J9, and R848-induced expression of TNF-α, IL-1β, and IL-17A were inhibited by JM17 (FIG. 4).

The inhibitory effect of J9 and JM17 on animal model with imiquimod (IMQ)-induced psoriatic inflammation.

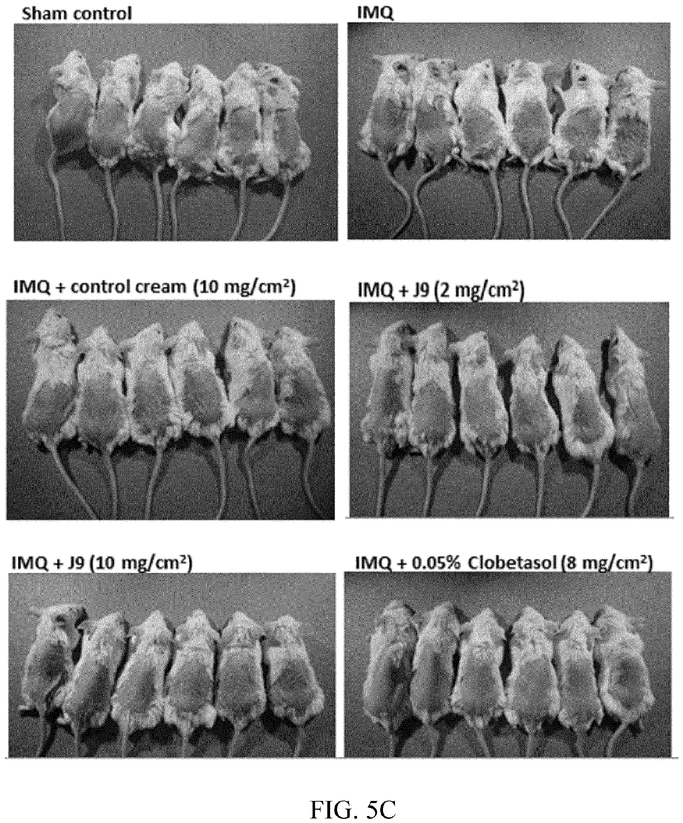

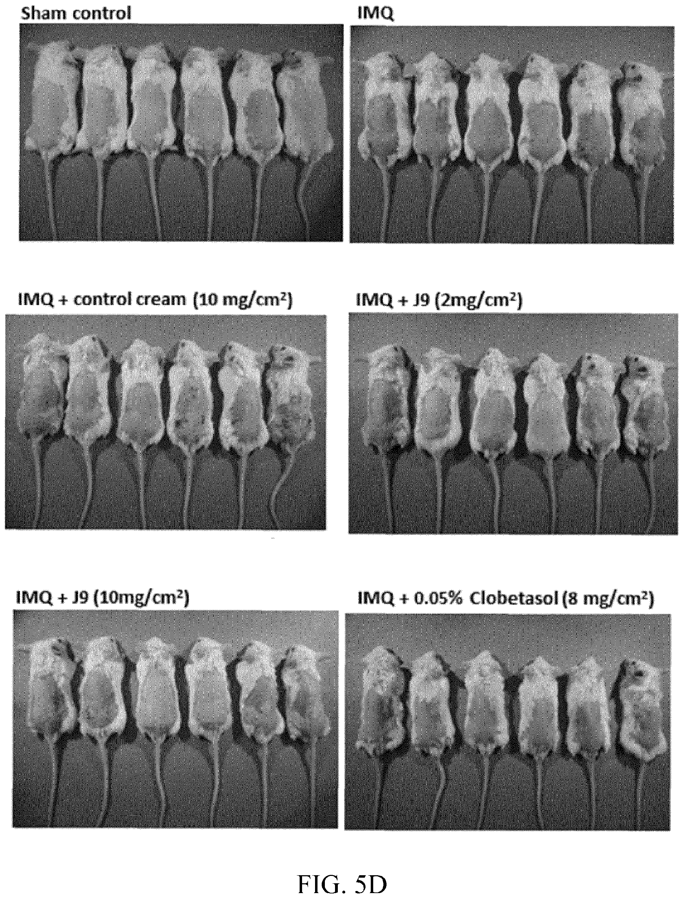

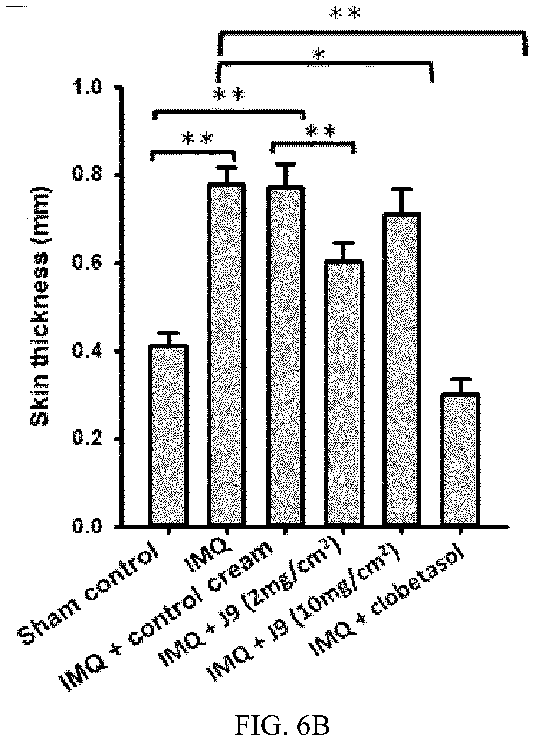

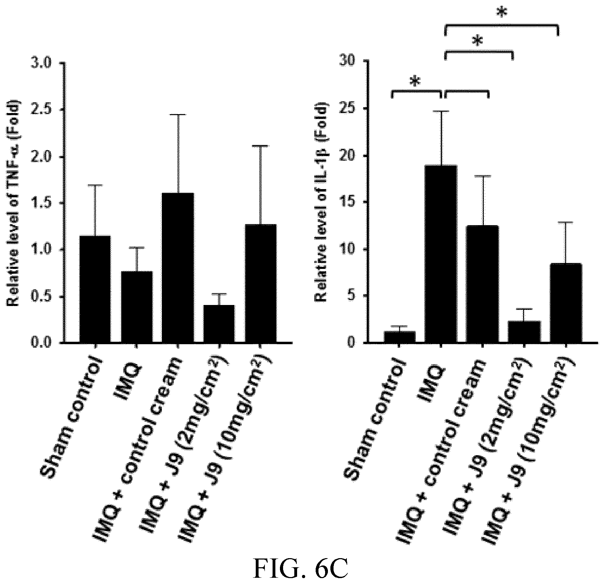

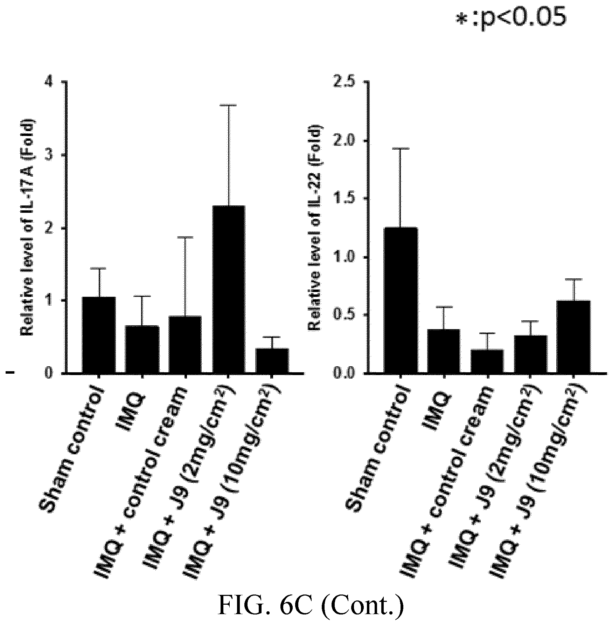

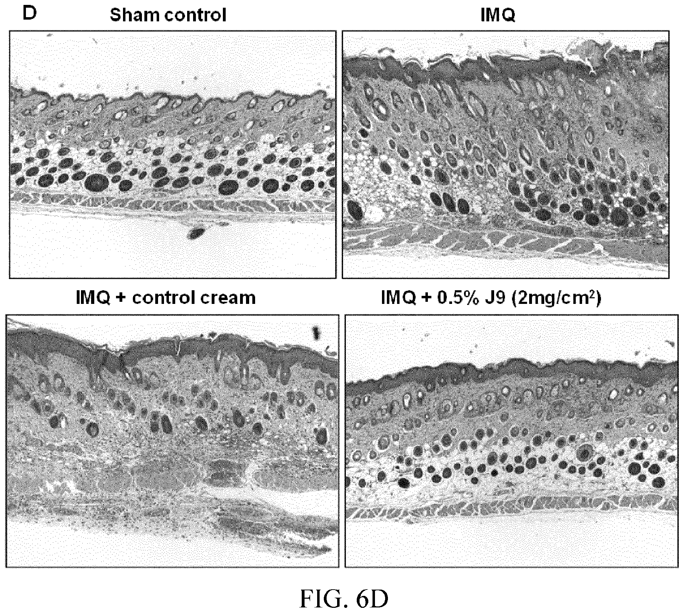

We further evaluated anti-inflammatory effects of J9 on psoriatic inflammation *in vivo.* First, we compared the effects of J9 control cream, J9 cream and clobetasol on inhibition of IMQ-induced psoriatic inflammation in a 10-day experimental schedule (FIG. 5A). Mice of each group were photoed for evaluation at day 0, day 5 and day 10 (FIGs. 5B, 5C and 5D). After 5 day treatment, lesional skins of IMQ treated group were observed with severely erythema, scaling and increase of skin thickness (FIG. 5B). In control cream group, there were no inhibitory effects observed (FIG. 5B). Treatment of J9 (2 mg/cm² and 10 mg/cm²) significantly reduced erythema, scaling, and thickness in lesional skins (FIG. 5B). In addition, treatment with corticosteroid drug clobetasol strongly inhibited IMQ-induced erythema, scaling and increase of skin thickness in lesional skin (FIG. 5B). However, clobetasol treatment markedly reduced skin thickness (FIGs 5B and 6B). In this experiment, we found that the severity of erythema, scaling and increase of skin thickness did not increase at day 10 compared to these parameters at day 5. These phenomena were reached most severe at day 5 (FIG. 5 and 6A). Psoriasis Area Severity Index (PASI) score of these groups were compared and we found that PASI score in mice group treated 2 mg/cm² of J9 were significantly decreased at day 3 to day 10. In mice group treated 10 mg/cm² of J9 were significantly decreased at day 4 to day 6 (FIG. 6A). The skin thickness were measured at day 10. Treatment of J9 (2 mg/cm² and 10 mg/cm²) significantly inhibited IMQ-induced increase of thickness (FIG. 6B). We also found that treatment of clobetasol reduced skin thickness compared to sham control group (FIG. 6B). Expression of cytokine genes including TNF-α, IL-1β, IL-17A, IL-22, IL-23 and IL-31 in lesional skin were also analyzed. Of these cytokines, we observed that J9 attenuated IMQ-induced IL-1β expression at day 10 (FIG. 6C). H&E staining results revealed that treatment of J9 reduced thickeness of the epidermis and dermis and keratinocyte proliferation in epidermis and inflammation (FIG. 6D).

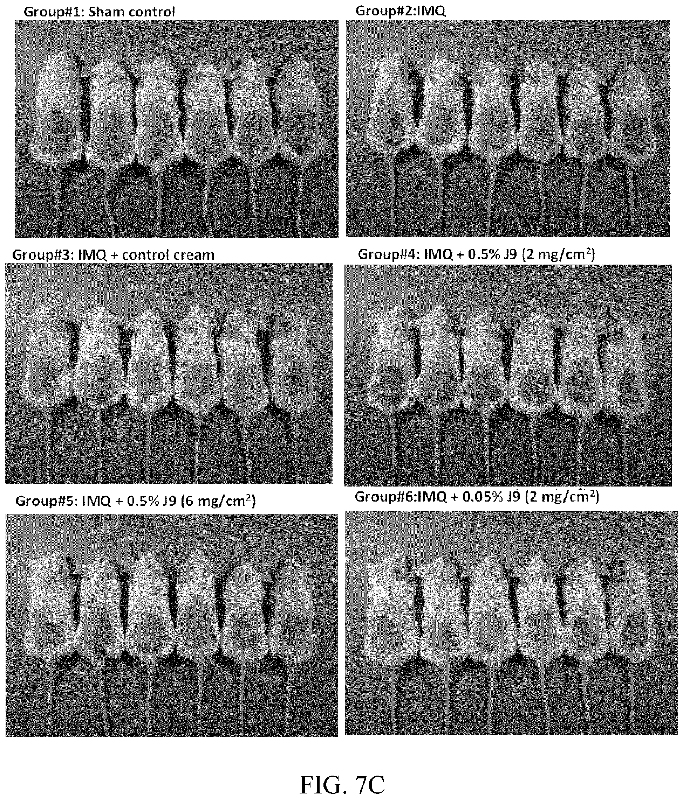

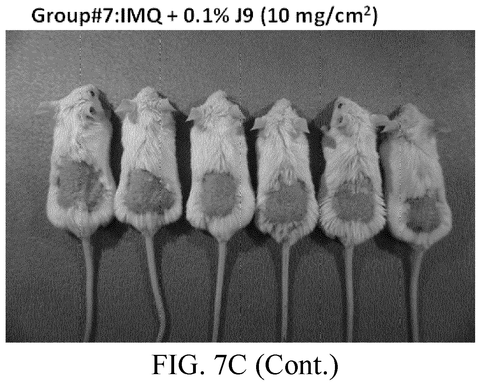

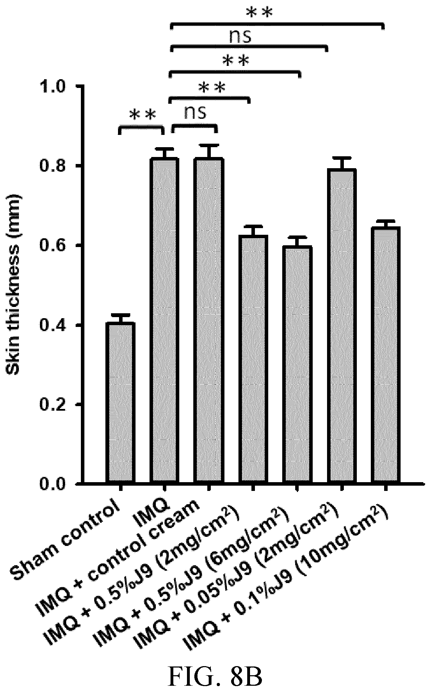

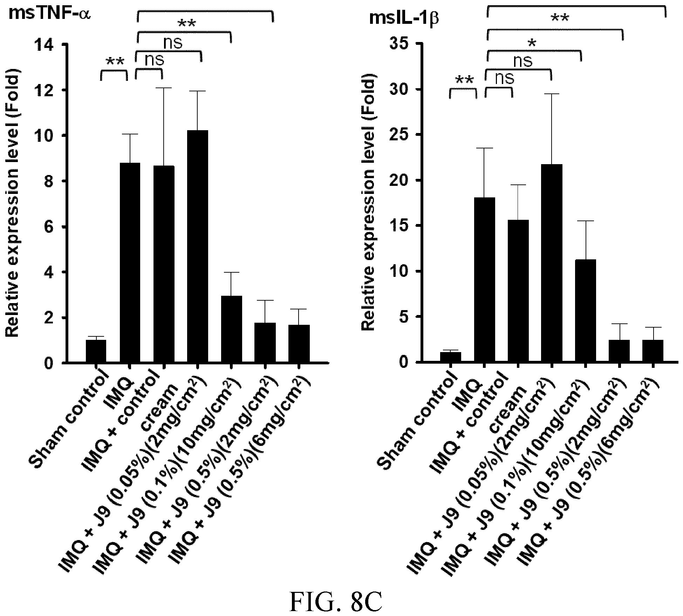

To further understand the therapeutic effects of different concentration of J9 cream, 0.5% J9 (2 mg/cm² and 6 mg/cm²), 0.1% J9 (10 mg/cm²) and 0.05% J9 cream(2 mg/cm²) were used to evaluate therapeutic effects on IMQ-induced psoriatic inflammation in a five-day experimental schedule (FIG. 7A). Mice of each group were photoed for evaluation at day 0 and day 5 (FIGs. 7B and 7C). After 5 day treatment, we found that IMQ-induced inflammation were inhibited in mice treated with 0.5% J9 (2 mg/cm² and 6 mg/cm²) and 0.1% J9 (10 mg/cm²) but not in mice treated with 0.05% J9 cream(2 mg/cm²) (FIG. 7C). PASI score, skin thickness and gene expression of lesional skin were also measured. PASI scores of mice treated with 0.5% J9 (2 mg/cm² and 6 mg/cm²) and 0.1% J9 (10 mg/cm²) were marked decreased at day 4 to day 5 (FIG. 8A). The similar results were also observed in skin thickness (FIG. 8B). IMQ-induced expression of TNF-α and IL-1β in lesional skin were also inhibited in mice treated with 0.5% J9 (2 mg/cm² and 6 mg/cm²) and 0.1% J9 (10 mg/cm²) (FIG. 8C). We also analyzed the induction and inhibition of IL-17A, IL-22, IL-23 and IL-31 in the lesional skin by IMQ and J9 in this experiment but significant results were not obtained because the expression level of these cytokines were low.

### Example 2: Pruritus

Two keloid patient derived fibroblasts named 256K and 268K were treated with ASC-J9 for 24 hr. The cells were then harvested and mRNA expression of ARTN and GAPDH were determined by qPCR.

As shown in FIG. 9, ASC-J9 suppresses pruritus markers in keloid patient derived fibroblasts. ASC-J9 suppresses pruritus through down-regulating artemin (ARTN) expression in patient derived keloid fibroblasts.

While the present invention has been described in conjunction with the specific embodiments set forth above, many alternatives thereto and modifications and variations thereof will be apparent to those of ordinary skill in the art. All such alternatives, modifications and variations are regarded as falling within the scope of the present invention.

## Claims

1. A pharmaceutical composition for use in a method for treating an inflammatory disorder relating to an inflammatory cytokine overexpression in a subject in need of such treatment, comprising administrating to said subject the pharmaceutical composition comprising an effective amount of a compound according to formula V or VIII and optionally a pharmaceutically acceptable carrier or excipient; 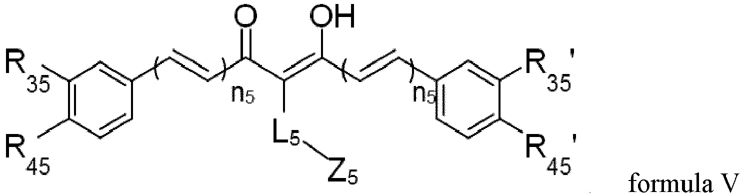 wherein in formula V:
each "ns" is independently 1, 2, or 3;
R₃₅, R₄₅, R₃₅, and R₄₅' are independently selected from the group consisting of -H, - OH, and -OCH₃;
L₅-Z₅ is absent; or
L₅-Z₅ exists, and L₅ is selected from the group consisting of a C0-C8 alkylene, an unsaturated alkenylene, and alkynl when Z₅ is absent;
Z₅ is selected from the group consisting of -H, -OH, an aromatic ring, a cycloalkyl, - COR₁₅, -CO₂R₁₅, -CONR₁₅R₂₅, -NR₁₅R₂₅, -C(X₅)_{3;}
R₁₅ and R₂₅ are independently selected from the group consisting of -H, -CH₃, and - C₂H₅; and
X₅ is a halogen atom selected from the group consisting of -F, -Cl, and -Br; 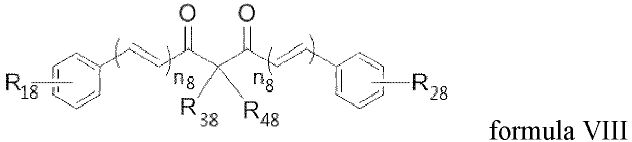
R₁₈ and R₂₈ are mono- or di-substituted groups and independently selected from the group consisting of a methoxy group, a hydroxyl group, and an alkyl sulfonyl group;
R₃₈ is selected from the group consisting of 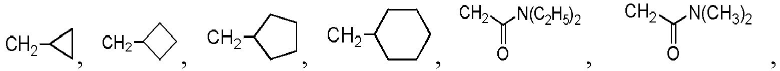 (CH₂)₃CH₃, 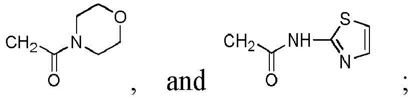 and
R₄₈ is selected from the group consisting of CH₃, H, F and Cl; and
n₈ is 1 or 2;
wherein the inflammatory cytokine is selected from the group consisting of INF-α, IL-1β, IL-17A, IL-22 and IL-31.

2. The method according to claim 1, wherein the compound is formula V, and "n₅" is 1; R₃₅, R₄₅, R₃₅', and R₄₅' are -OCH₃; and L₅-Z₅ side chain is absent.

3. The pharmaceutical composition for use in a method for treating an inflammatory disorder according to claim 1, wherein the compound is formula VIII, R₁₈ and R₂₈ are di-substituted methoxy groups, R₃₈ is 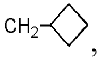 R₄₈ is H, and n₈ is 1.

4. The pharmaceutical composition for use in a method for treating an inflammatory disorder according to at least one of the preceding claims, wherein the pharmaceutical composition is a topical pharmaceutical composition.

5. The pharmaceutical composition for use in a method for treating an inflammatory disorder according to claim 4, wherein the pharmaceutical composition further comprises an emollient, skin penetrant, emulsifying agent, neutralizing agent, gelling agent, preservative, or chelating agent.

6. The pharmaceutical composition for use in a method for treating an inflammatory disorder according to at least one of the preceding claims, wherein the inflammatory cytokine is synthesized by dendritic cells or macrophages.

7. The pharmaceutical composition for use in a method for treating an inflammatory disorder according to at least one of the preceding claims, wherein the disorder is an epidermal disorder.

8. The pharmaceutical composition for use in a method for treating an inflammatory disorder according to at least one of claims 1 to 6, wherein the disorder is a dermal disorder.

9. The pharmaceutical composition for use in a method for treating an inflammatory disorder according to at least one of claims 1 to 6, wherein the disorder is an inflammatory skin disorder.

10. The pharmaceutical composition for use in a method for treating an inflammatory disorder according to claim 9, wherein the disorder is actinic keratosis, atopic dermatitis, chickenpox, cold sore, contact dermatitis, eczema, hives, impetigo, keratosis pilaris, latex allergy, measles, psoriasis, ringworm, rosacea, keloids, hypertrophic scars, pruritus, or seborrheic eczema.

11. The pharmaceutical composition for use in a method for treating an inflammatory disorder according to claim 10, wherein the disorder is psoriasis.

12. The pharmaceutical composition for use in a method for treating an inflammatory disorder according to claim 10, wherein the disorder is pruritus.

13. The pharmaceutical composition for use in a method for treating an inflammatory disorder according to at least one of claims 1 to 6, wherein the disorder is an autoimmune disease.

14. The pharmaceutical composition for use in a method for treating an inflammatory disorder according to claim 13, wherein the autoimmune disease is Addison's disease, celiac disease, graft versus host disease (GvHD), Graves' disease, Hashimoto's thyroiditis, inflammatory bowel diseases, multiple sclerosis, myasthenia gravis, pernicious anemia, psoriatic arthritis, rheumatoid arthritis (RA), Sjögren's syndrome, systemic lupus erythematosus (lupus), type 1 diabetes, or vasculitis.

15. The pharmaceutical composition for use in a method for treating an inflammatory disorder according to claim 14, wherein the inflammatory bowel diseases are ulcerative colitis or Crohn's disease.
